Europäisches Patentamt

European Patent Office

Office européen des brevets

(11) Publication number: **0 461 322 A1**

# EUROPEAN PATENT APPLICATION

(21) Application number: 90401643.3

(22) Date of filing: **13.06.90**

(51) Int. Cl.⁵: **C07C 51/10**, C07C 67/36,
C07C 69/84, C07C 65/105,
C07C 65/11

(43) Date of publication of application:
**18.12.91 Bulletin 91/51**

(84) Designated Contracting States:
**DE FR GB NL**

(71) Applicant: **Bromine Compounds Ltd.**
**Makleff House P.O.B. 180**
**Beer-Sheva 84101(IL)**

(72) Inventor: **Eisenstadt, Amichai**
**9 Henrieta Sold Street**
**Ramat Hasharon(IL)**

(74) Representative: **Gillard, Marie-Louise et al**
**Cabinet Beau de Loménie 55, Rue**
**d'Amsterdam**
**F-75008 Paris(FR)**

(54) **Method for the manufacture of hydroxy aromatic monocarboxylic acids.**

(57) A method for preparing hydroxy aromatic carboxylic acids, or the ester derivatives thereof, comprises the carbonylation of a hydroxy aromatic bromide in the presence of a catalytic amount of a transition element of group VIII of the Periodic Table on a carbon support, at a pH of above 8, in the absence of any promoter. The reaction is carried out at a temperature in the range of between 140°C to 200°C. The resulting products are obtained in high yields and of high purity. The method has particular applicability to the manufacture of 6-carboxy-2-naphthol and 4-hydroxy-4'-carboxy-biphenyl.

EP 0 461 322 A1

The present invention relates to a method for the manufacture of carboxylic acids and esters thereof by carbonylation of organic halides. More particularly, the invention relates to an improved method for the manufacture of hydroxy aromatic monocarboxylic acids and esters thereof by carbonylation of a corresponding hydroxy aromatic halide.

The term carbonylation as utilized in the present specification, means the introduction of a $C = O$ group into an organic molecule between the aromatic moiety and a functional moiety attached thereto.

The concept of carbonylation of organic halides in the presence of certain specific catalysts is known.

A well known process consists in the reaction of halogenated hydrocarbons with carbon monoxide and steam over active carbon, or silica gel at elevated temperatures, but the corresponding aromatic carboxylic acids are obtained in low yields. A large number of patents deal with variations of this approach in order to improve the yield and purity of the resultant products.

Thus, according to U.S. Patent Number 3,733,354 carboxylated aromatic compounds are obtained by the reaction of an aromatic halide with carbon monoxide and a compound containing an activated hydrogen in the presence of nickel tetracarbonyl and an organic solvent at a temperature in the range of 20°C to 130°C. Thus, for instance, using bromobenzene, dimethylsulfoxide, nickeltetracarbonyl, and triethylamine at 100°C, a mixture of benzoic acid and methyl benzoate was obtained. The total yield of the carboxylated products is claimed to be 96% with respect to the converted starting product. The use of nickelcarbonyl as catalyst is however disadvantageous considering its high volatility and toxicity.

The carbonylation of hydroxy aromatic compounds is disclosed in U.S Patent Nos. 3,769,324 and 3,769,326, said carbonylation occurring at the site of the hydroxy group. The reaction occurs between an aromatic alcohol and carbon monoxide in the presence of an iridium, osmium or ruthenium catalyst system at a temperature in the range of 50°C-300°C and carbon monoxide pressure in the range of 1-15,000 psi.

According to U.S. Patent Number 3,700,729 an oxidation reaction occurs on an aromatic compound with carbon monoxide in the presence of an anhydrous organic liquid reaction medium containing a catalyst, giving an oxidatively carbonylated aromatic compound which can be subsequently hydrolysed to form the corresponding carboxylic acid.

It has also been proposed in European Patent Application Numbers 206,958 and 255,794 to perform the carbonylation of aromatic halides in the presence of palladium catalysts with phosphinic ligands either in a homogeneous phase or in a phase-transfer system.The use of phosphinic ligands make this process economically disadvantageous.

European Patent Number 049,616 describes the preparation of naphtholic carboxylic acids by the carbonylation of halonaphthol in the presence of a reactive alcohol solvent having from 1 to 8 carbon atoms and a catalytic amount of complexes of a metal selected from palladium, platinum, ruthenium, rhodium, osmium or indium. The reaction occurs at a temperature in the range of 75°C to 200°C under a pressure in the range from 1 to 2000 psi. Yields in the range of 33% to 52% are said to be obtained.

An interesting process is described in a very recent U.S. Patent Number 4,654,436 claiming the manufacture of aromatic carboxylic acid esters.According to the process, an aromatic halide is reacted with carbon monoxide in the presence of a palladium catalyst promoted with a group VIB metal carbonyl and an alcohol at a pressure in the range of about 7 to 100 psi and at a temperature in the range of about 50°C to 180°C. Conversions of about 83% are obtained while in a comparative example described therein, performed under the same reaction conditions, but in the absence of the carbonyl promoter, only a very poor conversion of 20% to the desired product resulted. It should be pointed out that the use of phenols is not described at all. Furthermore, the use of higher temperatures which might be required for more resistant halocarbons, is said to be deleterious.

It is an object of the present invention to provide a method for the manufacture of hydroxy aromatic carboxylic acids and esters thereof. It is another object of the present invention to provide a simple method for the manufacture of hydroxy aromatic acids and esters thereof at high conversion and purity of the respective products. It is yet another object of the present invention to provide a simple method for the manufacture of biphenyl derivatives of high purity.

## BRIEF DESCRIPTION OF THE INVENTION

The invention relates to a simple method for the manufacture of hydroxy aromatic monocarboxylic acids by the carbonylation of a hydroxy aromatic bromide in the presence of a catalytic amount of a transition element of group VIII of the Periodic Table on a carbon support, at a pH of above 8 in the absence of any promoter.

When the corresponding ester of the hydroxy aromatic monocarboxylic acid is desired, a reactive alcohol having up to eight carbon atoms should also be incorporated.

It was surprisingly found that this carbonylation system produces very high conversions generally of above 98% and of purities of above 96%. The temperature required in order to achieve the above results is very critical and should be in the range of 140°C to 200°C and most preferably in the range of 160°C - 175°C.

The reaction is conducted at atmospheric pressure or slightly above i.e. up to 7 atmospheres, to enable an increase in the solubility of the carbon monoxide in the reaction system.

The preferred hydroxy aromatic bromide to be used has the general formula Br-Ar-OH, wherein Ar represents a carbocyclic or aromatic group, including biphenyl derivatives, having about 6 to 12 carbon atoms in the ring, or rings thereof. It should be pointed out that the preparation of hydroxy-carboxy biphenyl using the carbonylation method according to the present invention,is unique; alternative methods for the preparation of hydroxy aromatic acids (such as the Kolbe-Schmidt route) are not useful in the system using a halobiphenyl.

One may conceivably also use other halides,such as chlorides instead of bromides, but in this case the yields and purity will be lower than with the corresponding bromides. Typical examples of the radical groups Ar are: naphthalenes,substituted arenes,diphenyl ether, biphenyl, pyridine, etc. The substituents on the arene can be halide, hydroxyl, ether group, amino and dialkyl amino, carbamate groups or the like.

The reactive alcohol to be incorporated, in the case that an ester is to be obtained is an aliphatic alcohol such as pentanol, hexanol, 2-ethyl-1-hexanol, etc. Generally, the alcohol will also fulfil the function of a solvent for the reaction.

In some particular cases, additional inert solvents may be employed. Typical examples of such solvents are tetrahydrofuran, acetonitrile, etc.

The medium in which the reaction is carried out should be in the alkaline range preferably at a pH in the range of 8 to 11. The alkaline reagent to be introduced can be either an organic or inorganic base such as: sodium hydroxide, calcium oxide, potassium or sodium carbonate, tertiary amines such as triethylamine, etc. The amount of the alkaline reagent depends on the amount of the hydroxy aromatic substrate used in the reaction. Generally, this amount should be in the range of 1:1 to 1:2 (molar ratio) of hydroxy aromatic bromide to alkaline reagent. The carbonylation reaction occurs upon the introduction of the gaseous carbon monoxide into the reaction system, in amounts which provide a total reaction pressure in the range of 1 to 7 atmospheres and most preferably in the range of 1.5 to 5 atmospheres.

The carbonylation of the hydroxy aromatic bromide in accordance with the present invention is conducted in the presence of a catalytic amount of a group VIII metal catalyst. Any metal catalyst from this group, known to be catalytically active for carbonylation reactions such as those disclosed in the publication of A. Schoenberg et al (Journal of Organic Chemistry, Vol. 39, 3318-26, 1974) and U.S. Patents 4,016,194 and 4,034,004 may be used. It is most preferred that the catalyst be selected from palladium and iridium metal on a support consisting of an inert material such as carbon.Generally,the concentration of the metal catalyst on the carbon support is beween 2% to 10% by weight and preferably 3 to 7% by weight. The order of mixing of the reactants is not critical and can be varied depending on the equipment employed.

Complete reaction generally requires from 2 to 12 hours depending on the temperature and the amount of catalyst. When an inert solvent (or a reactive alcohol) is present during the reaction, the solvent - or the excess of reactive alcohol - as well as the unreacted hydroxy aromatic halide can be removed from the reaction system by distillation, and reused in a subsequent carbonylation reaction. Also, the catalyst recovered at the end of the reaction can be reused after washing.The hydroxy aromatic acid product (or its ester, when an alcohol is present) is obtained in a high degree of purity.

The method according to the present invention can be carried out in batch, semi-continuous or continuous operation.

The carbonylation method according to the present invention is applicable to many hydroxy aromatic bromides obtaining hydroxy aromatic monocarboxylic acids - or esters thereof, when a reactive alcohol is present - according to the particular aromatic bromide utilized. Examples of such hydroxy aromatic monocarboxylic acids and esters thereof are: n-butyl p-hydroxybenzoate; 2-ethyl-1-hexyl p-hydroxybenzoate, 2-hydroxy-6-naphthoic acid, 4-hydroxy-4'-carboxy-biphenyl, etc.

Summing up, the method according to the present invention provides an efficient, novel and economical method for producing specific high purity hydroxy aromatic acids or esters thereof. The most prominant factors which enable the performance of the method, to obtain high yields of pure products are as follows:

(a) The absence of a promoter such as mentioned in the prior art but operating at higher temperatures.

(b) Carrying out the reaction at a relatively high temperature in the range of 140°C to 200°C.

(c) Use of a very reactive catalyst such as a transition element of group VIII of the Periodic Table on an inert carbon support.

The invention will be further described with reference to the following Examples, although it will be understood that these Examples are included merely for purposes of illustration and are not intended to limit the scope of the invention and that many variations which include modifications and embodiments may be envisaged as being covered by the appended Claims.

In the Examples, the concentrations are given in weight per cent unless otherwise stated.

## EXAMPLE 1.

Preparation of n-butyl p-hydroxybenzoate.

Into a 100cc Fisher-Porter pressure bottle were placed: 1.7 g p-bromophenol (10 mmoles), 1.1 g $Na_2CO_3$, 39 mg 10% Pd/C and 17 ml n-butanol. The flask was pressurized twice with CO at 45 psi and immediately vented, then charged with CO to a pressure of 3 Atm. The reaction mixture was stirred vigorously at 150°C for 17 hrs. At that stage, the measured pressure had dropped to 2.4 atm. The conversion to butyl p-hydroxybenzoate was 58%.

## EXAMPLE 2.

Preparation of 2-ethyl-1-hexyl p-hydroxybenzoate.

Into a four-necked flask equipped with gas-bubbler, condenser, thermometer (thermocouple well) and septum port for sampling, were added: 5.96 g p-bromophenol (34 mmoles), 2.81g sodium carbonate, 110mg 4,5% Pd/C and 60ml (50g) 2-ethyl-1-hexanol. CO was bubbled in beneath the surface of the vigorously stirred mixture maintained at 157-160°C, at a flow rate of 20ml/min., for 4.5 hrs. The conversion to 2-ethyl-1-hexyl p-hydroxybenzoate was 97.5% (based on the p-bromophenol employed). The excess octanol was stripped off from the reaction mixture, after filtration of the Pd/C and sodium bromide, under reduced pressure. The oily residue was hydrolysed in an aqueous ethanol-NaOH mixture, followed by acidification with 37% hydrochloric acid to give a quantitative yield of p-HBA of a high degree of purity.

## EXAMPLE 3.

Preparation of 6-carboxy-2-naphthol (CHN).

A four-necked flask was charged with: 16.4g of 6-bromo-2-naphthol (0.074 moles), 6.33g $Na_2CO_3$ (0.06 moles), 351.4mg Pd/C (3.2%) and 120 ml 2-ethyl-1-hexanol. Nitrogen was flushed through the system for 20 mins., then replaced by CO at atmospheric pressure (20 ml/min. flow). The carbonylation process was carried out at 160°C for 7 hrs. with stirring whereupon the conversion of 6-bromo-2-naphthol to the 2-ethyl-1-hexyl ester of 2-hydroxy-6-naphthol acid was 96.2%, and only 0.3% of starting material was detected.

After the reaction, the flask was cooled and vented. 50cc water was added to the reaction mixture, which was then filtered in order to remove the heterogeneous catalyst. The excess of octanol was distilled from the organic layer. The product, a viscous oil, was dissolved in 80cc 95% ethanol. 8.9 g NaOH in 40 ml water was added, and the mixture was refluxed for 2.5 hrs. The hydrolysis mixture was allowed to cool and the ethanol was stripped off by distillation followed by the addition of 40cc water. The resulting 2-phase system was separated. The aqueous layer was further extracted with ethyl acetate (20ml) to remove all traces of organic materials, then acidified with aqueous HCl (37%), to bring the pH to 3.0-3.5. The heavy white-grey precipitate was filtered, washed with water and dried in an oven under vacuum to give 12.6 g of 2-hydroxy-6-naphthoic acid (90.3%). Crystallization from hot water and acetone yielded a white solid with a melt-. ing of of 250°C.

The above ester, as well as the acid, were characterised by IR, NMR and Gas Chromatography-Mass Spectrometry.

## EXAMPLE 4.

Preparation of 6-carboxy-2-naphthol.

The palladium-on-charcoal used in the carbonylation process of Example 3, was recovered by filtration, washing with water and ethyl acetate followed by drying at room temperature under water-pump vacuum. The recovered catalyst was reused for a second alkoxycarbonylation of 6-bromo-1-naphthol (BHN).

5.12g BHN (0.0231M), 2.16g $Na_2CO_3$, 45ml 2-ethyl-1-hexanol and the recovered Pd/C from Example 3, were reacted under the same conditions as mentioned therein.

A conversion of 94% to the ester was observed after 4.5 hrs. and 96% after 6 hrs.

## EXAMPLE 5.

Preparation of 6-carboxy-2-naphthol.

The catalyst used in Example 4 was recovered, washed with water and ethyl acetate and reused directly in a third reaction mixture, using the following reagents: 6 g BHN (0.027M), 2.27 g $Na_2CO_3$, 48cc 2-ethyl-1-hexanol, under the same conditions. Gas Chromatography analysis showed 92.4% conversion by weight to the desired ester after 7 hrs. reaction and 95% after 8 hrs.

## EXAMPLE 6.

Preparation of 4-hydroxy-4'-carboxy-biphenyl - (HCBP).

The same reactor was used as in the Example 3.

To the reactor were added 4.96 g of 4-hydroxy-4'-bromobiphenyl (HBBP,20 mmoles), 1.66 g Na₂CO₃, 0.105 g of 4.5% palladium on carbon, and 50 cc 2-ethyl-1-hexanol. After flushing the mixture with nitrogen for 10 minutes, a stream of carbon monoxide was fed beneath the surface of the stirred mixture at a flow of 47 ml/min. The reaction mixture was heated at 160°C for 16 hrs. An additional amount of catalyst (0.36 g) was added 9 hrs after CO feeding started; GC analysis then showed 83% of the 2-ethyl-1-hexyl ester of 4-hydroxy- 4'-carboxy-biphenyl (HCBP).

The reaction mixture was allowed to cool to room temperature and then vented with air, 15 cc water were added under vigorous stirring; the 2-phase system was filtered and separated from the solid catalyst. The organic layer was separated and the excess 2-ethyl-1-hexanol was stripped at 75°C/l mm. The viscous residue was hydrolysed in 45 cc refluxing 95% alcohol,1.4 g NaOH and 10 ml water for 2.5 hrs.A white precipitate was obtained which, after drying, gave 2.8g (65%), melting point >294°C. (literature 294.5°C), with a purity of 93%.

## Example 7.

Multicycled alkoxycarbonylation of HBBP.

Five batches of HCBP were prepared, recycling the Pd/C and the ethylhexanol each time.The Pd/C from the previous cycle was washed with water and HCl (1:1), water and acetone, then dried and used on a fresh batch of HBBP along with 20% make-up Pd/C. The alcohol was distilled from the reaction mixture and reused for the next batch. 0.1 M of substrate was used in each cycle. The combined residues (after the ethylhexanol was removed) were subjeted to alkaline hydrolysis in ethanol, to give the solid salt of the HCPB.

Throughout the five cycles, an amount of 2.89 g of 4.8% Pd/C (1.31 mmoles of Pd metal) was used (corresponding to a HBBP/Pd ratio of 381).

Work-up of the hydrolysis process after HCl treatment gave 81-83% yield with 95.5% purity. Purification of the crude product was performed by crystallization from acetone/water mixture.

## Claims

1.  A method for the manufacture of hydroxy aromatic monocarboxylic acids by the carbonylation of a hydroxy aromatic bromide in the presence of a catalytic amount of a transition element of group VIII of the Periodic Table on a carbon support, at a pH of above 8, in the absence of any promoter.

2.  The method according to Claim 1, wherein the carbonylation is carried out at a temperature in the range of 140°C to 200°C.

3.  The method according to Claims 1 and 2, carried out at a pressure in the range of 1 to 7 atmospheres.

4.  The method according to Claims 1 to 3, wherein the hydroxy aromatic bromide has the general formula Br-Ar-OH wherein Ar represents a carbocyclic or aromatic hydrocarbon having about 6 to 12 carbon atoms in the ring or rings thereof.

5.  The method according to Claims 1 to 4, carried out in an inert solvent as medium of reaction.

6.  The method according to Claims 1 to 5, wherein said hydroxy aromatic bromide is p-bromophenol, producing n-butyl p-hydroxybenzoate.

7.  The method according to Claims 1 to 5, wherein said hydroxy aromatic bromide is 6-bromo-2-naphthol, producing 6-carboxy-2-naphthol.

8.  The method according to Claims 1 to 5, wherein said hydroxy aromatic bromide is 4-hydroxy-4'-bromobiphenyl producing 4-hydroxy-4'-carboxy-biphenyl.

9.  The method according to Claims 1 to 5, carried out in the presence of a reactive alcohol as medium of reaction, producing the corresponding ester of the hydroxy aromatic monocarboxylic acid.

10. The method according to Claims 1 to 5, carried out at a pH in the range of 8 to 11.

11. The method according to Claim 10, wherein said pH is achieved by performing the reaction in the presence of an organic or inorganic base.

12. The method according to Claim 11 wherein said inorganic base is selected from sodium hydroxide, calcium oxide, potassium or sodium carbonate, calcium oxide, potassium carbonate, sodium carbonate or any mixture thereof.

13. The method according to Claim 11, wherein said organic base is selected from tertiary amines.

14. The method according to Claims 12 or 13, wherein the amount of organic or inorganic base is between 1:1 to 1:2 (molar ratio) of hydroxy aromatic bromide to said basic reagent.

15. The method according to Claim 1, wherein the transition element of group VIII of the Periodic Table is selected from palladium and iridium.

16. The method according to Claim 1, wherein the concentration of the metal catalyst on the carbon support is between 2 to 10% by weight.

17. The method according to Claim 1, wherein at the end of the reaction the catalyst is washed and reused in a subsequent carbonylation reaction.

18. A method for the manufacture of ester of hydroxy aromatic monocarboxylic acids and acids therefrom substantially as described in the specification and claimed in any one of Claims 1 to 17.

19. Hydroxy aromatic monocarboxylic acids substantially as prepared according to any one of Claims 1 to 17.

European
Patent Office

EUROPEAN SEARCH
REPORT

Application Number

EP 90 40 1643

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 049 616   (CELANESE CORP.)<br>* page 15, line 8; claims 1-6, 13, 17 *<br>- - - | 1,9-11,<br>13,15 | C 07 C 51/10<br>C 07 C 67/36<br>C 07 C 69/84<br>C 07 C 65/105 |
| X | EP-A-0 206 543   (I.C.I)<br>* col. 14, example 14 *<br>- - - | 1,2,5,6,<br>11,12 | C 07 C 65/11 |
| A | PATENT ABSTRACTS OF JAPAN vol. 12, no. 40<br>(C-474)(2887) 05 February 1988,<br>& JP-A-62 190145 (ASAHI) 20 August 1987,<br>* the whole document *<br>- - - | 1 | |
| A | World Patent Index database, Derwent Publ. Ltd London,<br>GB. Accession no. 77-27865Y week 16,1977<br>& JP-A-52-29891 (Teijin) (07.03.77)<br>- - - - - | 1 | |

TECHNICAL FIELDS
SEARCHED (Int. Cl.5)

C 07 C

The present search report has been drawn up for all claims

| Place of search | Date of completion of search | Examiner |
|---|---|---|
| Berlin | 15 February 91 | PROBERT C.L. |